# EUROPÄISCHE PATENTSCHRIFT

(11) **EP 2 417 910 B1**
(45) Veröffentlichungstag und Bekanntmachung des Hinweises auf die Patenterteilung: **26.06.2013**
(21) Anmeldenummer: 10172469.8
(22) Anmeldetag: 11.08.2010
(51) Int. Cl.: A61B 5/15

(54) **Analytische Testeinheit und Testsystem**
Analytical test unit and test system
Unité de test analytique et système de test

(43) Veröffentlichungstag der Anmeldung: 15.02.2012
(73) Patentinhaber: Roche Diagnostics GmbH, 68305 Mannheim (DE); F. Hoffmann-La Roche AG, 4070 Basel (CH)
(72) Erfinder: List, Hans, 64754 Hesseneck-Kailbach (DE); Harttig, Herbert, 67434 Neustadt (DE); Aigner, Simon, 69115 Heidelberg (DE)
(74) Vertreter: Pfiz, Thomas

(56) Entgegenhaltungen:
- EP-A1- 2 103 256
- WO-A2-2004/085995
- WO-A2-2005/006985
- US-A1- 2007 038 149

## Beschreibung

Die Erfindung betrifft eine analytische Testeinheit zum Gebrauch in einem Testgerät für den Nachweis eines Analyten in einer Körperflüssigkeit, insbesondere für Blutzuckertests, mit mindestens einem vorzugsweise in einem Magazin bereitgestellten Testelement, das eine Trägerfolie und eine auf einer Trägerseite der Trägerfolie aufgebrachte, mit der Körperflüssigkeit beaufschlagbare Reagenzschicht aufweist, wobei die lichtdurchlässige Trägerfolie zur optischen Abtastung der Reagenzschicht im Strahlengang einer photometrischen Messeinheit positionierbar ist. Die Erfindung betrifft weiter ein Testsystem zur Verarbeitung derartiger Testeinheiten.

Derartige analytische Testeinheiten werden beispielsweise in tragbaren Blutzuckermessgeräten eingesetzt, um dem Benutzer eine Selbstbestimmung des Blutzuckerspiegels in einem weitgehend automatischen Messablauf zu ermöglichen. Dabei wird die Reagenzschicht vorderseitig mit einer Blutprobe benetzt und rückseitig durch die Trägerfolie hindurch photometrisch vermessen. Die gängigste Methode zur optischen Auswertung besteht darin, unter einem Winkel zwischen 30° und 60° auf die Folienoberfläche Licht einzustrahlen und das remittierte Licht senkrecht zur Oberfläche mit einem Photodetektor aufzufangen. Alternativ können die Positionen von Beleuchtung und Detektion vertauscht werden. Zweck dieser Anordnungen ist, direkte Reflektionen an Oberflächen des Testaufbaus aus dem Detektionsstrahlengang herauszuhalten. Solche Reflektionen ergeben eine sehr große Signalhöhe am Detektor, haben aber nicht mit der Testchemie interagiert und enthalten deshalb keine Information über den Analyten, der die Testchemie optisch verändert. Außerdem bewirken schon geringste Winkeländerungen zwischen Lichtein- bzw. Lichtausfall und spiegelnder Oberfläche einen großen Signalhub im detektierten Licht, was von den Modulationen der Remission aufgrund des Analyten nicht unterschieden werden kann. Ein solcher Strahlengang benötigt außerdem einen größeren Bauraum, der bei Integration von Testelementen in einen Probensammler, der die Probenflüssigkeit durch Hauteinstich gewinnen soll, zumindest dann nicht vorhanden ist, wenn viele solcher integrierten Verbrauchsmittel auf engem Raum magaziniert werden sollen.

Ausgehend hiervon liegt der Erfindung die Aufgabe zugrunde, die im Stand der Technik bekannten Testeinheiten und Systeme weiter zu verbessern und insbesondere für kompakte Handgeräte optimierte optische Messwerterfassung mit hoher Messgenauigkeit zu ermöglichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen. Eine analytische Testeinheit gemäß dem Oberbegriff des Anspruchs 1 ist aus Dokument US 20070038149 bekannt.

Die Erfindung geht von dem Gedanken aus, glatte spiegelnde Grenzflächen im Testaufbau durch eine topologische Oberflächenkontur zu vermeiden. Dementsprechend wird erfindungsgemäß vorgeschlagen, dass die Trägerfolie eine durch eine erhabene bzw. dreidimensional feinstrukturierte Oberflächenstruktur modifizierte Oberfläche zur Verminderung von Reflexionen im Strahlengang der Messeinheit aufweist. Unter "feinstrukturiert" ist dabei zu verstehen, dass die Strukturelemente um mehrere Größenordnungen kleiner als die modifizierte Oberfläche selbst sind. Auf diese Weise ist möglich, störende Reflexionen durch Entspiegelung oder Lichtumlenkung aus dem Strahlengang der Messeinheit fernzuhalten. Dies kann selbst bei flächennormaler Einstrahlung erreicht werden, so dass eine besonders raumsparende direkte optische Ankopplung ermöglicht wird.

Vorteilhafterweise ist die Oberflächenstruktur dazu ausgebildet, den Brechungsindex der Trägerfolie in Richtung der Flächennormale kontinuierlich zu ändern. Alternativ kann die Oberflächenstruktur eine Mikrooptik zur Auslenkung störender Reflexionen aus dem Strahlengang der Messeinheit bilden.

Eine weitere vorteilhafte Ausgestaltung sieht vor, dass die Oberflächenstruktur auf der Trägerseite und/oder auf der davon abgewandten und zu der Messeinheit hin ausgerichteten Rückseite der Trägerfolie angeordnet ist.

Gemäß einer besonders bevorzugten Variante ist die Oberflächenstruktur auf der Trägerseite angeordnet und als insbesondere periodisches Oberflächenrelief nach Art einer Mottenaugenstruktur ausgebildet. Durch eine solche Mottenaugenstruktur, wie sie beispielsweise zur Entspiegelung von optischen Datenträgern an sich bekannt ist, kann eine kontinuierliche Änderung des Brechungsindexes der Trägerfolie zu der Reagenzschicht hin bewirkt werden. Es gibt also keine definierte optische Grenzfläche zwischen Trägerfolie und Reagenzschicht und damit auch keine Spiegelung.

Durch eine solche Ausgestaltung lässt sich auch eine intrinsische Brechungsindexanpassung erreichen, wenn der Brechungsindex der Reagenzschicht durch Benetzung mit Körperflüssigkeit im Zuge der photometrischen Messwerterfassung sich zeitlich ändert.

Um die Antireflexeigenschaften zu optimieren, sollte die Oberflächenstruktur eine Strukturhöhe im Bereich zwischen dem 5-fachen und 0,2-fachen, vorzugsweise zwischen dem 3-fachen und 0,7-fachen, bevorzugt zwischen dem 2-fachen bis 1-fachen der Wellenlänge des Messlichts der Messeinheit besitzen. Damit lässt sich auch erreichen, dass der Transmissionsgrad der Trägerfolie durch die Oberflächenstruktur im Vergleich zu einer unstrukturierten ebenen Oberfläche erhöht wird.

Eine weitere besonders vorteilhafte Ausführung sieht vor, dass die Oberflächenstruktur durch ein Prismenprofil auf der von der Trägerseite abgewandten Rückseite der Trägerfolie gebildet ist. Auf diese Weise lässt sich der Messstrahlengang durch Lichtbrechung beeinflussen, um störende Reflexe auszuleiten.

Vorteilhafterweise ist das Prismenprofil mit einer Profilteilung von weniger als 100 µm, vorzugsweise weniger als 50 µm aus einer Vielzahl von Einzelprismen gebildet. Damit lässt sich eine hinreichende Mittelung über einen Lichtspot speziell am Austrittsende eines Lichtleiters erreichen und zugleich eine hinreichend definierte Anlagefläche für die optische Ankopplung schaffen.

Eine weitere Verbesserung wird dadurch erreicht, dass das Prismenprofil durch ein in einer Längsrichtung gerade verlaufendes und quer dazu periodisches Dreieckprofil, insbesondere ein Sägezahnprofil gebildet ist.

Die Oberflächenstrukturen können als vorzugsweise heißgeprägte, von einem Prägewerkzeug abgeformte Prägestruktur in die Trägerfolie eingebracht sein. Alternativ ist es auch möglich, die genannten Oberflächenstrukturen durch eine insbesondere mittels eines Formwerkzeugs formgebend ausgehärtete Gießschicht zu bilden.

Um den Messablauf und die Handhabung weiter zu vereinfachen, ist es vorteilhaft, wenn eine mit der Reagenzschicht in fluidischer Verbindung stehende oder bringbare Sammelstruktur für Körperflüssigkeit, insbesondere eine an einem Stechelement angeordnete Kapillare zum Gewinnen von Körperflüssigkeit durch einen Hauteinstich als Baueinheit integriert ist.

Gegenstand der Erfindung ist auch ein analytisches Testsystem zum Nachweis eines Analyten in einer Körperflüssigkeit, insbesondere als tragbares Handgerät für Blutzuckertests, enthaltend eine photometrischen Messeinheit und mindestens ein im Strahlengang der Messeinheit positionierbares Testelement, das eine lichtdurchlässige Trägerfolie und eine auf einer Trägerseite der Trägerfolie aufgebrachte, mit der Körperflüssigkeit beaufschlagbare Reagenzschicht aufweist, wobei die Trägerfolie eine durch eine dreidimensional geformte Oberflächenstruktur modifizierte Oberfläche zur Verminderung von Reflexionen im Strahlengang der Messeinheit aufweist. In einem solchen Testsystem lassen sich die erfindungsgemäßen Testeinheiten mit den vorgenannten Merkmalen besonders vorteilhaft einsetzen.

Um eine kompakte optische Schnittstelle zu schaffen, ist es besonders bevorzugt, wenn die Messeinheit mehrere Lichtleiter zur Übertragung von Messlicht aufweist, wobei die Lichtleiter an einem Stirnende an die von der Reagenzschicht abgewandte Rückseite der Trägerfolie vorzugsweise auf Stoß angekoppelt sein können.

Dabei haben sich parallele Anordnungen von Beleuchtungs- und Detektionslichtleitern als besonders tauglich erweisen, um auf kleinstem Raum (beispielsweise innerhalb nur eines Kubikmillimeters) eine optische Schnittstelle aufzubauen. Da solche Lichtleiter auch aus Gründen der Herstellbarkeit parallel verlaufen sollten, liegt ein Lichtweg vor, auf dem Reflexe von allen Grenzflächen, die nicht direkt auf den Enden der Lichtleiter aufliegen, in den Detektor gelangen könnten. Um dies zu vermeiden, ist es besonders vorteilhaft, wenn die Lichtleiter zumindest mit ihren auf die Trägerfolie ausgerichteten Endabschnitten in einer gemeinsamen Ebene parallel zueinander angeordnet sind, und wenn die Oberflächenstruktur durch ein periodisches Prismenprofil gebildet ist, welches mit seinem Profilquerschnitt senkrecht zu der Ebene der Lichtleiter steht. Durch eine solche Anordnung wird einfallendes Licht geeignet gebrochen, so dass eine direkte Reflexion in den Empfangslichtleiter vermieden wird. Dabei ist es günstig, wenn der seitliche Versatz des abgelenkten Lichts größer als der Durchmesser der Lichtleiter ist.

Zur Erhöhung des Gebrauchskomforts für den Benutzer ist es vorteilhaft, wenn eine Vielzahl von Testelementen in der Testeinheit magaziniert sind. Die Testeinheit kann dabei als Drehmagazin für einzelne integrierte Probensammler (Microsampler) oder als Bandkassette zur sukzessiven Bereitstellung von Testelementen auf einem Transportband ausgebildet sein.

Im Folgenden wird die Erfindung anhand der in der Zeichnung schematisch dargestellten Ausführungsbeispiele näher erläutert. Es zeigen
- Fig. 1: ein analytisches Blutzucker-Testsystem mit einer darin eingesetzten disposiblen Testeinheit in einer perspektivischen Ansicht;
- Fig. 2: einen Ausschnitt des Testsystems mit einem an ein Testelement der Testeinheit angekoppelten Lichtleiterstößel;
- Fig. 3: eine vereinfachte perspektivische Darstellung der Anordnung nach Fig. 2 in der das Testelement eine rückseitige Prismenstruktur zur Ankopplung der Lichtleiter aufweist;
- Fig. 4: eine ausschnittsweise Ansicht quer zu der Ebene der Lichtleiter nach Fig. 3 mit einem symbolisierten Strahlengang;
- Fig. 5: eine weitere Ausführungsform einer Testeinheit mit reflexmindernden Mottenaugenstruktur in einer Seitenansicht;
- Fig. 6: eine perspektivische Darstellung eines Testträgers der Testeinheit nach Fig. 5.

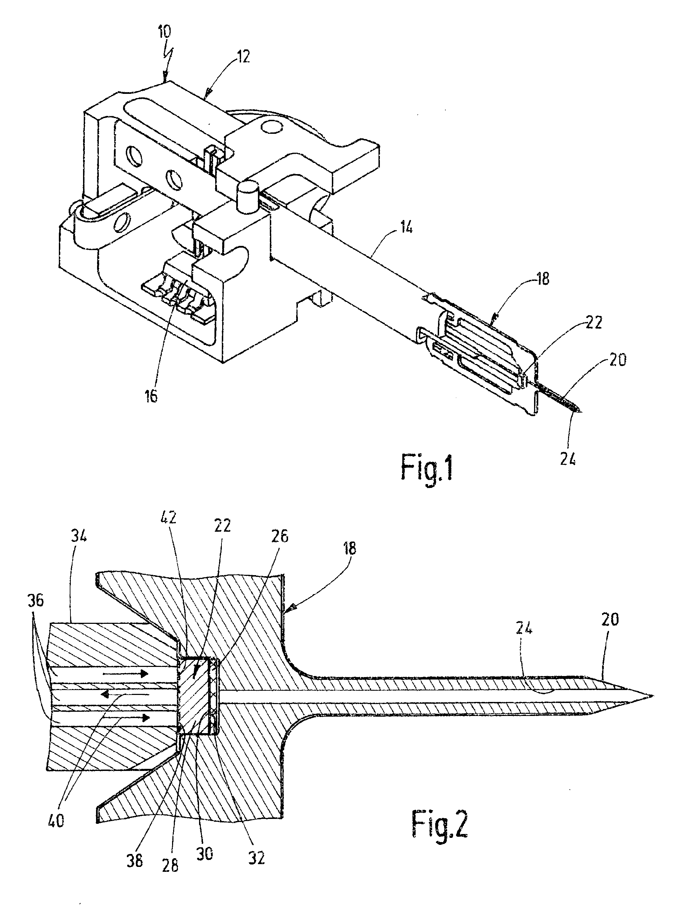

Das in Fig. 1 dargestellte Testsystem 10 umfasst einen Geräteteil 12 mit vor und zurück gehendem Stechantrieb 14 und einer photometrischen Messeinheit 16 in einem nicht gezeigten Gehäuse als tragbares Handgerät und darin einsetzbare analytische Testeinheiten 18 mit Stechelement 20 und integriertem Testelement 22 für einen Einmaltest an einer Flüssigprobe, speziell zur Glukosebestimmung in einer Blutprobe. Weitere Details der Anordnung beispielsweise hinsichtlich der Bereitstellung von Testeinheiten aus einem Drehmagazin ergeben sich auch aus der EP09159834.2, auf die in diesem Zusammenhang ausdrücklich Bezug genommen wird.

Wie auch aus Fig. 2 ersichtlich, ist das Stechelement 20 mit einer Kapillarrille 24 versehen, die das bei einem Hauteinstich beispielsweise aus einem Finger eines Probanden gewonnene Blut vorderseitig auf eine saugfähige Spreitschicht 26 des integrierten Testelements 22 leitet. Dieses besitzt eine Trägerfolie 28 und eine auf deren Trägerseite 30 unter der Spreitschicht 26 eingeschlossene trockenchemische Reagenzschicht 32, die bei Benetzung mit der Körperflüssigkeit irreversibel durch eine Farbänderung auf den Analyten reagiert. Dies lässt sich rückseitig durch die transparente Trägerfolie 28 hindurch erfassen. Zu diesem Zweck weist die Messeinheit 16 einen als Stößel in den Stechantrieb 14 eingebauten Optikadapter 34 auf, der drei parallel verlaufende Lichtleiter 36 enthält, die an ihren freien Stirnflächen auf Stoß mit der Rückseite 38 der Trägerfolie 28 in Anlage bringbar sind. Dabei sind die Lichtleiter 36 in Richtung der Flächennormalen ausgerichtet. Die äußeren Lichtleiter sind mit einem Lichtsender verbunden, während der innere Lichtleiter das an der Reagenzschicht 32 gestreute Messlicht auf einen Lichtempfänger der Messeinheit 16 zurückleitet, wie es in Fig. 2 durch Pfeile als Strahlengang 40 veranschaulicht ist.

Um störende Reflexionen im Strahlengang 40 weitgehend zu vermindern, ist die Trägerfolie 28 an ihrer Rückseite 38 und/oder Vorderseite 30 mit einer auf einem mikroskopischen Maßstab dreidimensional geformten Oberflächenstruktur 42 versehen, die einen stetigen Brechzahlverlauf bewirkt oder eine Optik zur Lichtauslenkung bildet.

In den Figuren 2 bis 4 ist eine solche optisch wirksame Oberflächenstruktur 42 in Form eines rückseitigen Prismenprofils 44 auf der Trägerfolie 28 gezeigt. Dieses Prismenprofil 44 verläuft parallel zu einer Seite des rechteckigen Trägerfolienstücks 28 geradlinig und weist quer dazu ein periodisches Sägezahnprofil 46 auf. Die Profilabmessungen sind so gewählt, dass auch Ungleichmäßigkeiten im Lichtdurchtritt der Lichtleiter 36 über deren Querschnitt ausgemittelt werden und eine zuverlässige Auflage gewährleistet ist. Bei einem Lichtleiterdurchmesser von 125 µm ist ein Prismenprofil 44 mit einer Profilteilung von 25 bis 30 µm gut geeignet. Damit wird eine hinreichende Mittelung über den Lichtleiterquerschnitt erreicht, während die Profilstruktur noch groß genug ist, um die Herstellung zu ermöglichen.

Die Herstellung der Prismenstruktur kann beispielsweise durch spanabhebende Techniken erfolgen. Denkbar ist auch die Ausbildung einer Gießschicht auf der Trägerfolie 28, die mittels eines geeignet geformten Werkzeugs formgebend ausgehärtet wird.

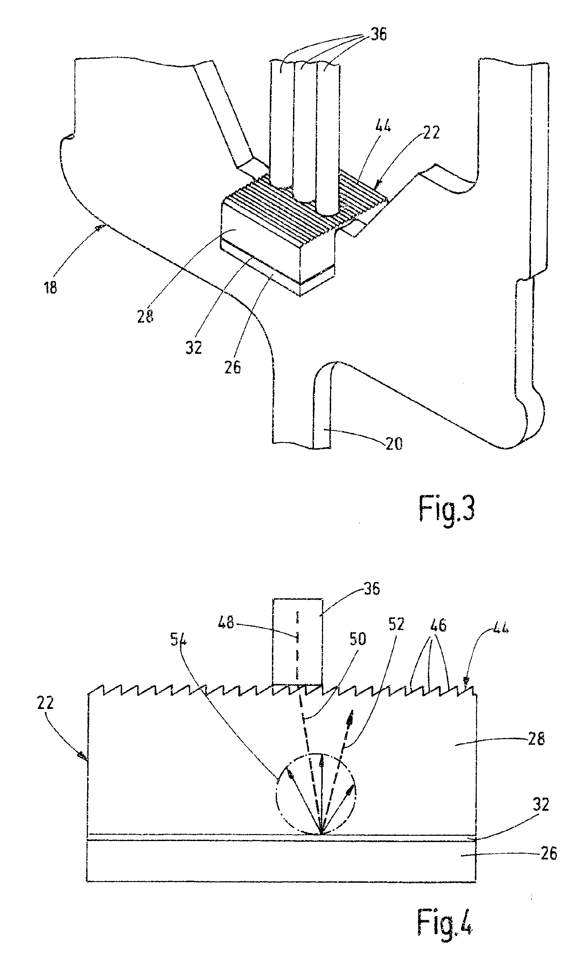

Die Lichtauslenkung durch das Prismenprofil 44 ist in Fig. 4 veranschaulicht. Die senkrecht zur Trägerfolie 28 auf der Profiloberfläche aufstehenden Lichtleiter 36 sind parallel zueinander in einer Ebene angeordnet, die rechtwinklig zu der gezeigten Profilquerschnittsfläche steht. Aufgrund des schrägen Sägezahnprofils 46 wird ein aus dem Lichtleiter 36 austretender Zentralstrahl 48 durch Brechung aus der Ebene der Lichtleiter 36 seitlich heraus abgelenkt (Teilstrahl 50). Ein Teil dieses Lichts wird an der Grenzfläche zu der Reagenzschicht 32 ohne Wechselwirkung mit der Testchemie regulär reflektiert. Dieser reflektierte Lichtstrahl 52 tritt im Winkel zu dem Einfallslot aus und kann somit nicht mehr in den Strahlengang der Messeinheit gelangen. Der Ablenkwinkel des Prismenprofils 44 sollte nach Maßgabe des Akzeptanzwinkels der Lichtleiter 36 und deren stirnseitigem Abstand zur reflektierenden Grenzfläche gewählt werden.

Nur derjenige Anteil des einfallenden Lichts 50, der in die Reagenzschicht 32 gelangt und von dort als diffuse Lichtkeule 54 zurückgestreut wird, kann zum Teil über den Empfangslichtleiter zur Messeinheit 16 gelangen. Dieses Messlicht ist somit in Wechselwirkung mit der Testchemie getreten und beinhaltet Information über den Analyten.

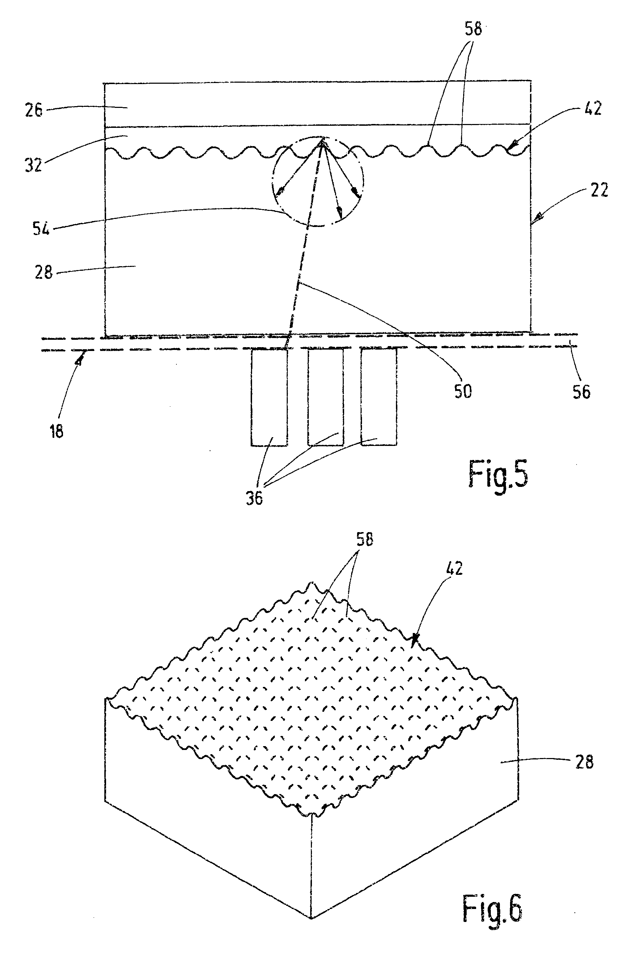

Fig. 5 und 6 zeigen ein Ausführungsbeispiel einer reflexmindernden Oberflächenstruktur 42, die auf der Trägerseite des durch die Trägerfolie 28 gebildeten Blocks die Reagenzschicht 32 trägt. Auch ein solches Testelement 22 kann in dem vorstehend beschriebenen Stechelement 20 integriert sein. Denkbar ist auch der Einsatz in einer Bandkassette als Testeinheit 18, die auf einem aufwickelbaren transparenten Transportband 56 eine Vielzahl von Testeinheiten 22 beinhaltet.

Die Oberflächenstruktur 42 weist in diesem Fall ein durch nicht maßstäblich dargestellte Erhebungen (und komplementäre Vertiefungen) gebildetes periodisches Oberflächenrelief 58 nach Art einer sogenannten "Mottenaugenstruktur" auf, wie sie beispielsweise in der EP-A 0 288 140 beschrieben ist. Das Einbringen dieser Oberflächenstruktur vermeidet eine definierte Grenzfläche und führt zu einer kontinuierlichen Änderung des Brechungsindexes zu der Reagenzschicht 32 hin, so dass eine effiziente Entspiegelung bewirkt wird. Wie in Fig. 5 der Einfachheit halber nur für einen Randstrahl 50 des aus dem Lichtleiter 36 austretenden Lichtkegels veranschaulicht, tritt an der Mottenaugenstruktur 58 praktisch keine Spiegelung auf, so dass der Transmissionsgrad der Trägerfolie 28 erhöht wird und im Wesentlichen nur die Rückstreuung 54 aus der Reagenzschicht 32 als Messlicht in den Empfangslichtleiter eintritt.

Bei einer schmalbandigen Anregung beispielsweise mit einer Messwellenlänge von 365 nm sollten die Erhebungen bzw. Vertiefungen der Mottenaugenstruktur 58 eine Strukturhöhe bzw. - tiefe im Bereich zwischen dem 5-fachen und 0,2-fachen, vorzugsweise zwischen dem 3-fachen und 0,7-fachen und bevorzugt zwischen dem 2-fachen bis 1-fachen der Messwellenlänge besitzen. Auch die laterale Strukturperiode sollte in der Größenordnung der Wellenlänge des Messlichts liegen. Solche nanoskalig feinstrukturierten Oberflächen lassen sich beispielsweise durch Heißprägen von einem Prägewerkzeug auf die Trägerfolie 28 abformen, wobei ggf. die Werkzeugoberfläche durch Ätzen hergestellt werden kann.

## Patentansprüche

1. Analytische Testeinheit zum Gebrauch in einem Testgerät für den Nachweis eines Analyten in einer Körperflüssigkeit, insbesondere für Blutzuckertests, mit mindestens einem Testelement (22), das eine Trägerfolie (28) und eine auf einer Trägerseite (30) der Trägerfolie (28) aufgebrachte, mit der Körperflüssigkeit beaufschlagbare Reagenzschicht (32) aufweist, wobei die lichtdurchlässige Trägerfolie (28) zur optischen Abtastung der Reagenzschicht (32) im Strahlengang einer photometrischen Messeinheit (16) positionierbar ist, **dadurch gekennzeichnet, dass** die Trägerfolie (28) eine durch eine erhabene Oberflächenstruktur (42) modifizierte Oberfläche zur Verminderung von Reflexionen im Strahlengang der Messeinheit (16) aufweist.

2. Analytische Testeinheit nach Anspruch 1, **dadurch gekennzeichnet, dass** die Oberflächenstruktur (42) den Brechungsindex der Trägerfolie (28) in Richtung der Flächennormale kontinuierlich ändert oder eine Optik zur Auslenkung störender Reflexionen aus dem Strahlengang der Messeinheit (16) bildet.

3. Analytische Testeinheit nach Anspruch 1 oder 2, **dadurch gekennzeichnet, dass** die Oberflächenstruktur (42) auf der Trägerseite (30) und/oder auf der davon abgewandten und zu der Messeinheit (16) hin ausgerichteten Rückseite der Trägerfolie (28) angeordnet ist.

4. Analytische Testeinheit nach einem der Ansprüche 1 bis 3, **dadurch gekennzeichnet, dass** die Oberflächenstruktur (42) auf der Trägerseite (30) angeordnet ist und ein insbesondere periodisches Oberflächenrelief (58) nach Art einer Mottenaugenstruktur bildet.

5. Analytische Testeinheit nach einem der Ansprüche 1 bis 4, **dadurch gekennzeichnet, dass** die Oberflächenstruktur (42;58) eine Strukturhöhe im Bereich zwischen dem 5-fachen und 0,2-fachen, vorzugsweise zwischen dem 3-fachen und 0,7-fachen, bevorzugt zwischen dem 2-fachen bis 1-fachen der Wellenlänge des Messlichts der Messeinheit (16) besitzt.

6. Analytische Testeinheit nach einem der Ansprüche 1 bis 5, **dadurch gekennzeichnet, dass** der Transmissionsgrad der Trägerfolie (28) durch die Oberflächenstruktur (42) im Vergleich zu einer unstrukturierten ebenen Oberfläche erhöht ist.

7. Analytische Testeinheit nach einem der Ansprüche 1 bis 3, **dadurch gekennzeichnet, dass** die Oberflächenstruktur (42) durch ein Prismenprofil (44) auf der von der Trägerseite (30) abgewandten Rückseite (38) der Trägerfolie (28) gebildet ist.

8. Analytische Testeinheit nach Anspruch 7, **dadurch gekennzeichnet, dass** das Prismenprofil (44) mit einer Profilteilung von weniger als 100 µm, vorzugsweise weniger als 50 µm aus einer Vielzahl von Einzelprismen (46) gebildet ist.

9. Analytische Testeinheit nach Anspruch 7 oder 8, **dadurch gekennzeichnet, dass** das Prismenprofil (44) durch ein in einer Längsrichtung gerade verlaufendes und quer dazu periodisches Dreieckprofil, insbesondere ein Sägezahnprofil gebildet ist.

10. Analytische Testeinheit nach einem der Ansprüche 1 bis 9, **dadurch gekennzeichnet, dass** die Oberflächenstruktur (42) als vorzugsweise heißgeprägte, von einem Prägewerkzeug abgeformte Prägestruktur in die Trägerfolie (28) eingebracht ist.

11. Analytische Testeinheit nach einem der Ansprüche 1 bis 9, **dadurch gekennzeichnet, dass** die Oberflächenstruktur (42) durch eine insbesondere mittels eines Formwerkzeugs formgebend ausgehärtete Gießschicht gebildet ist.

12. Analytische Testeinheit nach einem der Ansprüche 1 bis 11, **gekennzeichnet durch** eine mit der Reagenzschicht (32) in fluidischer Verbindung stehende oder bringbare Sammelstruktur (24) für Körperflüssigkeit, insbesondere eine an einem Stechelement (20) angeordnete Kapillare zum Gewinnen von Körperflüssigkeit **durch** einen Hauteinstich.

13. Analytisches Testsystem zum Nachweis eines Analyten in einer Körperflüssigkeit, insbesondere als tragbares Handgerät für Blutzuckertests, enthaltend eine photometrischen Messeinheit (16) und mindestens ein im Strahlengang der Messeinheit (16) positionierbares Testelement (22), das eine lichtdurchlässige Trägerfolie (28) und eine auf einer Trägerseite (30) der Trägerfolie (28) aufgebrachte, mit der Körperflüssigkeit beaufschlagbare Reagenzschicht (32) aufweist, **dadurch gekennzeichnet, dass** die Trägerfolie (28) eine durch eine dreidimensional geformte Oberflächenstruktur (42) modifizierte Oberfläche zur Verminderung von Reflexionen im Strahlengang der Messeinheit (16) aufweist.

14. Analytisches Testsystem nach Anspruch 13, **dadurch gekennzeichnet, dass** die Messeinheit (16) mehrere Lichtleiter (36) zur Übertragung von Messlicht aufweist, und dass die Lichtleiter (36) an einem Stirnende an die von der Reagenzschicht (32) abgewandte Rückseite der Trägerfolie (28) vorzugsweise auf Stoß angekoppelt sind.

15. Analytisches Testsystem nach Anspruch 14, **dadurch gekennzeichnet, dass** die Lichtleiter (36) zumindest mit ihren auf die Trägerfolie (28) ausgerichteten Endabschnitten in einer gemeinsamen Ebene parallel zueinander angeordnet sind, und dass die Oberflächenstruktur (42) durch ein periodisches Prismenprofil (44) gebildet ist, welches mit seinem Profilquerschnitt senkrecht zu der Ebene der Lichtleiter (36) steht.

## Claims

1. An analytic test unit for use in a test instrument for detecting an analyte in a bodily fluid, particularly for blood sugar tests, comprising at least one test element (22), which has a carrier film (28) and a reagent layer (32) which is applied to a carrier side (30) of the carrier film (28) and to which the bodily fluid can be applied, wherein the light-transmissive carrier film (28) can be positioned in the beam path of a photometric measuring unit (16) for optically scanning the reagent layer (32), **characterized in that** the carrier film (28) has a surface which is modified by a raised surface structure (42) in order to reduce reflections in the beam path of the measuring unit (16).

2. The analytic test unit as claimed in claim 1, **characterized in that** the surface structure (42) continuously changes the refractive index of the carrier film (28) in the direction of the surface normal or forms an optics for deflecting disturbing reflections out of the beam path of the measuring unit (16).

3. The analytic test unit as claimed in claim 1 or 2, **characterized in that** the surface structure (42) is arranged on the carrier side (30) and/or on the rear side of the carrier film (28) facing away therefrom and directed at the measuring unit (16).

4. The analytic test unit as claimed in one of claims 1 to 3, **characterized in that** the surface structure (42) is arranged on the carrier side (30) and forms an in particular periodic surface relief (58) in the manner of a moth's eye structure.

5. The analytic test unit as claimed in one of claims 1 to 4, **characterized in that** the surface structure (42; 58) has a structure height in the range between 5-times and 0.2-times, preferably between 3-times and 0.7-times, preferred to be between 2-times and 1-times the wavelength of the measurement light of the measuring unit (16).

6. The analytic test unit as claimed in one of claims 1 to 5, **characterized in that** the transmission factor of the carrier film (28) is increased compared to an unstructured plane surface as a result of the surface structure (42).

7. The analytic test unit as claimed in one of claims 1 to 3, **characterized in that** the surface structure (42) is formed by a prism profile (44) on the rear side (38) of the carrier film (28) facing away from the carrier side (30).

8. The analytic test unit as claimed in claim 7, **characterized in that** the prism profile (44) is formed with a profile pitch of less than 100 µm, preferably less than 50 µm, from a multiplicity of individual prisms (46).

9. The analytic test unit as claimed in claim 7 or 8, **characterized in that** the prism profile (44) is formed by a triangle profile, more particularly a saw-tooth profile, which extends in a straight line in a longitudinal direction and which is periodic transversely thereto.

10. The analytic test unit as claimed in one of claims 1 to 9, **characterized in that** the surface structure (42) is introduced into the carrier film (28) as a preferably hot stamped stamping structure formed by a stamping tool.

11. The analytic test unit as claimed in one of claims 1 to 9, **characterized in that** the surface structure (42) is formed by cast layer, more particularly cured in a formed fashion by means of a forming tool.

12. The analytic test unit as claimed in one of claims 1 to 11, **characterized by** a collection structure (24) for bodily fluid, which is or can be brought into fluidic connection with the reagent layer (32), more particularly a capillary, which is arranged on a piercing element (20), for obtaining bodily fluid by piercing the skin.

13. An analytic test system for detecting an analyte in a bodily fluid, more particularly as portable hand-held instrument for blood sugar tests, comprising a photometric measuring unit (16) and at least one test element (22) which can be positioned in the beam path of the measuring unit (16) and has a light-transmissive carrier film (28) and a reagent layer (32), which is applied to a carrier side (30) of the carrier film (28) and to which the bodily fluid can be applied, **characterized in that** the carrier film (28) has a surface which is modified by a three-dimensionally shaped surface structure (42) in order to reduce reflections in the beam path of the measuring unit (16).

14. The analytic test system as claimed in claim 13, **characterized in that** the measuring unit (16) has a plurality of optical waveguides (36) for transmitting measurement light and **in that** the optical waveguides (36) are preferably coupled end-to-end at one end face to the rear side of the carrier film (28) facing away from the reagent layer (32).

15. The analytic test system as claimed in claim 14, **characterized in that** the optical waveguides (36) are arranged parallel to one another in a common plane, at least with the end sections thereof directed at the carrier film (28), and **in that** the surface structure (42) is formed by a periodic prism profile (44), the profile cross section of which being perpendicular to the plane of the optical waveguides (36).

## Revendications

1. Unité de test analytique destinée à être utilisée dans un appareil de test pour la détection d'un analyte dans un fluide corporel, en particulier pour des tests de glycémie, comprenant au moins un élément de test (22) qui présente une feuille support (28) et une couche de réactif (32) déposée sur une face support (30) de la feuille support (28), qui peut recevoir le fluide corporel, la feuille support (28) translucide pouvant être positionnée dans le trajet du faisceau d'une unité de mesure photométrique (16) en vue de l'exploration optique de la couche de réactif (32), **caractérisée en ce que** la feuille support (28) possède une surface modifiée par une structure superficielle (42) en relief, servant à réduire les reflets gênants dans le trajet du faisceau de l'unité de mesure (16).

2. Unité de test analytique selon la revendication 1, **caractérisée en ce que** la structure superficielle (42) modifiie de manière continue l'indice de réfraction de la feuille support (28) dans le sens de la normale de surface ou forme une optique pour la déviation des reflets gênants hors du trajet du faisceau de l'unité de mesure (16).

3. Unité de test analytique selon les revendications 1 ou 2, **caractérisée en ce que** la structure superficielle (42) est agencée sur la face support (30) et/ou sur la face arrière opposée à celle-ci et orientée vers l'unité de mesure (16) de la feuille support (28).

4. Unité de test analytique selon l'une des revendications 1 à 3, **caractérisée en ce que** la structure superficielle (42) est agencée sur la face support (30) et forme un relief superficiel (58) notamment périodique à la manière d'une structure en forme des yeux de mites.

5. Unité de test analytique selon l'une des revendications 1 à 4, **caractérisée en ce que** la structure superficielle (42 ; 58) possède une hauteur de structure comprise entre 5 et 0,2 fois, de préférence entre 3 et 0,7 fois, de manière préférée entre 2 et 1 fois la longueur d'onde de la lumière de mesure de l'unité de mesure (16).

6. Unité de test analytique selon l'une des revendications 1 à 5, **caractérisée en ce que** le facteur de transmission de la feuille support (28) est augmenté grâce à la structure superficielle (42) par rapport à une structure plane non structurée.

7. Unité de test analytique selon l'une des revendications 1 à 3, **caractérisée en ce que** la structure superficielle (42) est formée par un profil prismatique (44) sur la face arrière (38) opposée à la face support (30) de la feuille support (28).

8. Unité de test analytique selon la revendication 7, **caractérisée en ce que** le profil prismatique (44), ayant un pas de profil de moins de 100 µm, de préférence de moins de 50 µm, est formé par une pluralité de prismes individuels (46).

9. Unité de test analytique selon les revendications 7 ou 8, **caractérisée en ce que** le profil prismatique (44) est formé par un profil triangulaire rectiligne dans une direction longitudinale et périodique transversalement à celle-ci, en particulier un profil en dent de scie.

10. Unité de test analytique selon l'une des revendications 1 à 9, **caractérisée en ce que** la structure superficielle (42) est pratiquée dans la feuille support (28) de préférence sous la forme d'une structure estampée à chaud, modelée par un outil d'estampage.

11. Unité de test analytique selon l'une des revendications 1 à 9, **caractérisée en ce que** la structure superficielle (42) est formée par une couche moulée durcie, en particulier façonnée au moyen d'un outil de formage.

12. Unité de test analytique selon l'une des revendications 1 à 11, **caractérisée par** une structure de collecte (24) de fluide corporel, qui est ou peut être mise en liaison fluidique avec la couche de réactif (32), en particulier un tube capillaire placé sur un élément d'incision (20) pour extraire du fluide corporel par une incision de la peau.

13. Système de test analytique pour la détection d'un analyte dans un fluide corporel, en particulier sous forme d'appareil portatif pour tests de glycémie, comprenant une unité de mesure photométrique (16) et au moins un élément de test (22) pouvant être positionné dans le trajet du faisceau de l'unité de mesure (16), qui présente une feuille support (28) translucide et une couche de réactif (32) déposée sur une face support (30) de la feuille support (28), qui peut recevoir le fluide corporel, **caractérisé en ce que** la feuille support (28) possède une surface modifiée par une structure superficielle (42) formée en trois dimensions, servant à réduire les reflets gênants dans le trajet du faisceau de l'unité de mesure (16).

14. Système de test analytique selon la revendication 13, **caractérisé en ce que** l'unité de mesure (16) comporte plusieurs guides de lumière (36) pour la transmission de lumière de mesure, et **en ce que** les guides de lumière (36) sont accouplées de préférence en aboutement à une extrémité frontale à la face arrière opposée à la couche de réactif (32) de la feuille support (28).

15. Système de test analytique selon la revendication 14, **caractérisé en ce que** les guides de lumière (36), au moins avec leurs parties d'extrémité orientées vers la feuille support (28), sont disposés parallèlement les uns aux autres dans un plan commun, et **en ce que** la structure superficielle (42) est formé par un profil prismatique périodique (44) qui avec sa section transversale de profil est perpendiculaire au plan des guides de lumière (36).
